# EUROPEAN PATENT SPECIFICATION

(11) **EP 0 805 150 B1**
(45) Date of publication and mention of the grant of the patent: **19.12.2001**
(21) Application number: 97105609.8
(22) Date of filing: 04.04.1997
(51) Int. Cl.: C07D 249/08, C07D 487/04

(54) **A method for preparing 1H-1,2,4-triazol-5-yl acetic acid compounds, and the novel 3-(4-tert-butylphenyl)-1H-1,2,4-triazol-5-yl acetic acid**
Verfahren zur Herstellung von 1H-1,2,4-triazol-5-yl-Essigsäure Derivaten, sowie die neue 3-(4-tert-butylphenyl)-1H-1,2,4-triazol-5-yl Essigsäure
Procédé de préparation de dérivés d'acide 1H-1,2,4-triazol-5-yl acétique, ainsi que nouvel acide 3-(4-tert-butylphényl)-1H-1,2,4-triazol-5-yl acétique

(30) Priority: 04.04.1996 JP 8268896
(43) Date of publication of application: 05.11.1997
(60) Divisional application: 98123055.0
(73) Proprietor: Fuji Photo Film Co., Ltd., Kanagawa-ken (JP)
(72) Inventor: Shimura, Yoshio, Minami-Ashigara-shi, Kanagawa-ken (JP); Shimada, Yasuhiro, Minami-Ashigara-shi, Kanagawa-ken (JP)
(74) Representative: Hansen, Bernd, Dr. Dipl.-Chem.

(56) References cited:
- EP-A- 0 714 892
- EP-A- 0 720 981
- JP-A- 6 172 357
- JP-A- 7 048 376

## Description

### FIELD OF THE INVENTION

The present invention relates to a method for producing 1H-1,2,4-triazol-5-yl acetic acid compounds that are useful as coupler intermediates for photography and as intermediates for medicines and agricultural chemicals.

### BACKGROUND OF THE INVENTION

WIth respect to the method for synthesizing 1H-1,2,4-triazol-5-yl acetic acids, synthetic examples can be found, for example, in IZV, Akad, Nauk, SSSR, Ser. Khim., 9,2075 (1990); Chem Pharm. Bull., 36,96 (1988), and JP-A ("JP-A" means unexamined published Japanese patent application) No. 172357/1994. However, because in the methods described in these publications, for example, the yield is low, the reaction procedure and post-treatment are complicated, and the reaction takes much time, it is difficult to obtain the particular compounds efficiently in a high yield, which means that the methods are unsatisfactory as industrial production methods. As a technique relatively similar to the present invention, a method is disclosed in JP-A No. 48376/1995. Although the method disclosed therein is improved in comparison with the above synthetic examples, however, for example, the method must use, as a raw material, a compound represented by formula (2) given below in a free form (in the formula, n = 0), and in Step 2, an aqueous NaOH solution as a base is used, in which the method is different from the present invention. Further, in the method disclosed therein the solvent is removed by distillation, making the procedure complicated, and the yield is low, which means that the method is unsatisfactory as an industrial production method. Therefore, there is strong need for the development of a method for synthesizing 1H-1,2,4-triazol-5-yl acetic acids in a high yield, wherein the reaction procedure and post-treatment are simple.

### SUMMARY OF THE INVENTION

Therefore, a first object of the present invention is to provide a method for producing 1H-1,2,4-triazol-5-yl acetic acid compounds in a manner convenient to practice and in a high yield.

Other and further objects, features and advantages of the invention will appear more fully from the following description.

### DETAILED DESCRIPTION OF THE INVENTION

The inventors of the present invention have studied intensively to attain the above objects and, as a result, have completed the present invention. That is, the present invention provides:
(1) A method for preparing a 1H-1,2,4-triazol-5-yl acetic acid compound, comprising reacting a compound represented by the below-given formula (1), wherein R1 represents a alkyl group or aryl group (which alkyl or aryl group may be substituted one in this invention), with a compound represented by the below-given formula (2), wherein R2 represents an alkyl group, R3 represents an alkyl group or aryl group, A represents an acid substance selected from the group consisting of HCl, HBr, HI, phosphoric acid, H₂SO₄, and p-toluenesulfonic acid, and n is 0, 0.5 or 1, in the presence of water, a water-soluble organic solvent, and CH₃COOM or a combination of NaOH and CH₃COOM, in which latter M represents an alkali metal (e.g. Na, K, Li), to produce a compound represented by the below-given formula (3), wherein R1 and R2 have the same meanings as those in formulas (1) and (2); and then adding M₂CO₃, wherein M has the same meaning as defined above, followed by heating, thereby obtaining a compound represented by the below-given formula (4), wherein R1 has the same meaning as defined in formulas (1) and (3): 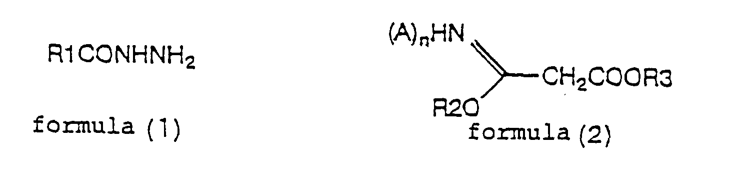 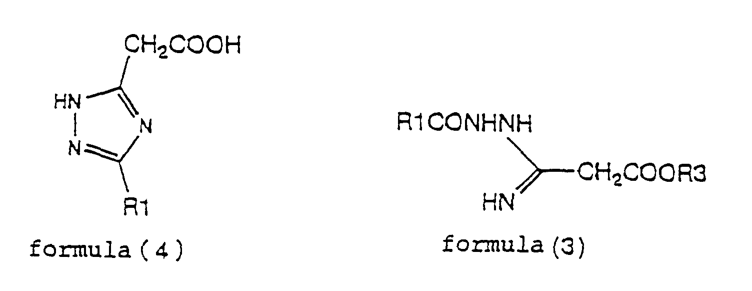
(2) A method for preparing a 1H-1,2,4-triazol-5-yl acetic acid compound, comprising heating a compound represented by formula (3) in the presence of water and M₂CO₃, wherein M has the same meaning as defined in the above (1), to obtain a compound represented by formula (4).
(3) The method for preparing the following 3-(4-tert-butylphenyl)-lH-1,2,4-triazol-5-yl acetic acids as stated in the above (1) or (2):

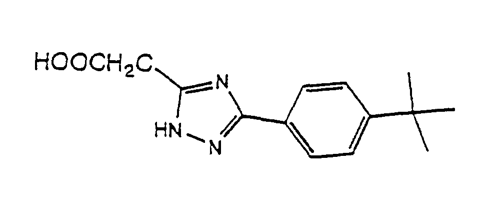

Among the 1H-1,2,4-triazol-5-yl acetic acid compounds, particularly 3-(4-tert-butylphenyl)-1H-1,2,4-triazol-5-yl acetic acid 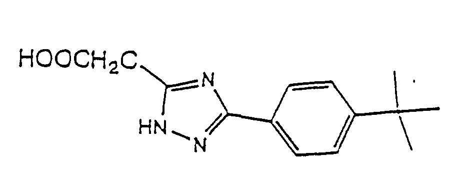 is a novel compound that has not yet appeared in the literature, and it is useful as a raw material for the synthesis of pyrrolotriazole cyan couplers that are excellent in coupling activity, heat stability, and light/heat fastness and hue of the dye image, which couplers are described in U.S. Patent Nos. 5,256,526 and 5,384,236. Further, it is advantageous that the raw material compound: p-tert-butylphenylhydrazide, corresponding to formula (1), which is a raw material for the synthesis of 3-(4-tert-butylphenyl)-lH-1,2,4-triazol-5-yl acetic acid, can be obtained very inexpensively through simple procedures in a high yield from 4-tert-butylbenzoic acid vinyl ester, which is known as a modifier for synthetic resins.

Next, the compounds represented by formulas (1), (2), and (3), that is, the raw materials of the present invention, and the compound represented by formula (4), that is, the intended compound, are described in detail. In formulas (1), (3), and (4), R1 is an optionally substituted monocyclic or condensed-ring aryl group that preferably has a total number of carbon (hereinafter abbreviated to carbon number) of 6 to 36, or it is an optionally substituted straight-chain, branched-chain, or cyclic alkyl group that preferably has a carbon number of 1 to 30, and examples of the substituents include a cyano group, a halogen atom (Cl, Br, F, I), an alkyl group, an alkoxy group, a carbonamido group, a sulfonamido group, an alkoxycarbonyl group, an aryloxycarbonyl group, an acyloxy group, and a nitro group.

In formulas (1), (3), and (4), more preferably R1 represents an optionally substituted aryl group having a carbon number of 6 to 30 (e.g. phenyl, 1-naphthyl, p-tolyl, o-tolyl, p-tert-butylphenyl, o-2-ethylhexyloxyphenyl, 4-methoxyphenyl, 4-methyl-3-nitrophenyl, 3,5-di-chlorophenyl, p-cyanophenyl, 2,4-di-tert-pentylphenyl, pentafluorophenyl, and p-methanesulfoneamidophenyl), or it is an optionally substituted alkyl group having a carbon number of 1 to 24 (e.g. methyl, isopropyl, tert-butyl, cyclohexyl, 2-ethylhexyl, 1,1,3,3-tetramethylbutyl, dodecyl, vinylmethyl, trifluoromethyl, methoxyethyl, ethoxycarbonylmethyl, and phenoxyethyl).

Particularly preferably, R1 represents an aryl group having a carbon number of 6 to 24 (e.g. phenyl, o-tolyl, p-tert-butylphenyl, 4-methyl-3-nitrophenyl, 3,5-di-chlorophenyl, and p-cyanophenyl), or an alkyl group having a carbon number of 1 to 20 (e.g. methyl, isopropyl, t-butyl, cyclohexyl, and phenoxyethyl), and most preferably it represents a 4-methyl-3-nitrophenyl group, a p-tert-butylphenyl, a tert-butyl group, a cyclohexyl group, or a phenoxyethyl group.

When R2 in formula (2) and R3 in formulas (2) and (3) are alkyl groups, the alkyl groups are independently alkyl groups that preferably have a carbon number of 1 to 10, and more preferably 1 to 4 (e.g. methyl, ethyl, and propyl). When R3s in formulas (2) and (3) are aryl groups, the aryl groups are independently aryl groups having a carbon number of 6 to 20, and more preferably 6 to 12 (e.g. phenyl, p-tolyl, and o-tolyl). Preferably R2 in formula (2) and R3 in formulas (2) and (3) are alkyl groups, and methyl and ethyl groups are particularly preferable.

The reaction of the present invention can be represented by the following scheme:

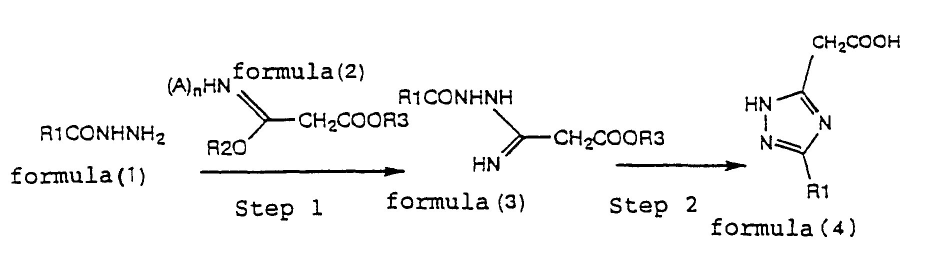

In Step 2, the ester group (-COOR3) in the compound of formula (3) is hydrolyzed upon a reaction in basic condition, to form an intermediate carboxylic acid. Then, the carboxylic acid formed from the compound of formula (3) is subjected to a ring-closure reaction, to form the compound of formula (4).

Now, how to carry out continuously Steps 1 and 2 stated in the above (1) is described in detail. The base used in Step 1 is NaOH or CH₃COOM, in which latter M represents an alkali metal, and examples of CH₃COOM are CH₃COOLi, CH₃COONa, and CH₃COOK, with particular preference given to CH₃COONa. Further, the amount of the base to be used (in a molar ratio to the compound represented by formula (1)) is from 0.8 to 10 : 1, preferably from 0.8 to 3.0 : 1, and particularly preferably from 1.0 to 1.5 : 1.

As the solvent used in Step 1, water and a water-soluble organic solvent (e.g. acetonitrile, tetrahydrofuran (THF), dioxane, MeOH, EtOH, isopropyl alcohol, acetone, and N,N-dimethylacetamide) can be mentioned, with preference given to acetonitrile and THF as the organic solvent, and particularly to acetonitrile. The amount of solvent (water + organic solvent) for 1 mol of the compound represented by formula (1) is 0.5 to 3.0 liters, and particularly preferably 0.5 to 1.5 liters. The amount of the compound represented by formula (2) (in a molar ratio to the compound represented by formula (1)) is from 0.8 to 1.5 : 1, preferably from 0.9 to 1.2 : 1, and particularly preferably from 1.0 to 1.1 : 1.

The reaction temperature is -30 to 80 °C, preferably -10 to 60 °C, and particularly preferably 0 to 30 °C. The reaction time is 1 min to 24 hours, preferably 5 min to 6 hours, and more preferably 5 min to 3 hours.

Next, Step 2 is described in detail. Examples of M₂CO₃ (wherein M represents an alkali metal), which is added to the reaction liquid containing the compound represented by formula (3) produced in Step 1, include Li₂CO₃, Na₂CO₃, and K₂CO₃, with preference given to Na₂CO₃ and K₂CO₃, and particularly to Na₂CO₃. The amount of M₂CO₃ (in a molar ratio to the raw material compound represented by formula (1) used in Step 1) is from 1 to 10 : 1, preferably from 1.5 to 5 : 1, and more preferably from 2 to 3 : 1. The amount of water added in Step 2 is 1 to 2 liters, and preferably about 1 liter, to 1 mol of the raw material compound represented by formula (1). The reaction temperature is 0 to 80 °C, preferably room temperature (20 °C) to 80 °C, and more preferably 65 to 80 °C. The reaction time is 30 min to 30 hours, and preferably 30 min to 2 hours.

The reaction in Step 2, wherein the compound represented by formula (3) stated under the above embodiment of (2) is used as a raw material compound, can be carried out under the same reaction conditions as in Step 2 stated under the above embodiment of (1). That is, the type of M₂CO₃ (wherein M represents an alkali metal) used in Step 2 is the same as that in the above (1), and the amount thereof (in a molar ratio to the raw material compound represented by formula (3)) is from 1 to 10 : 1, and preferably from 2 to 3 : 1. The amount of water to be added is 1 to 3 liters, and preferably about 2 liters, to 1 mol of the raw material compound represented by formula (3). The reaction temperature is 0 to 80 °C, preferably 20 to 80 °C, and more preferably 65 to 80 °C. The reaction time is 30 min to 3 hours, and preferably 30 min to 2 hours.

According to the present invention, 1H-1,2,4-triazol-5-yl acetic acid compounds that are useful as intermediates for photographic couplers, medicines, and agricultural chemicals can be obtained simply in a high yield.

The 3-(4-tert-butylphenyl)-1H-1,2,4-triazol-5-yl acetic acid compound gives a coupler which can form by a coupling reaction with an oxide of a color developing agent a dye that excels in hue and fastness, other dyes formed from a coupler obtained from a 3-phenyl-1H-1,2,4-triazol-5-yl acetic acid compound. Further, it is advantageous that the 3-(4-tert-butylphenyl)-1H-1,2,4-triazol-5-yl acetic acid compound can be synthesized at lower cost since a starting material, a compound of formula (1) (Rl: p-tert-butylphenyl) can be synthesized from a p-tert-butylbenzoic acid vinyl ester that is easily available as a polymer modifying agent.

### EXAMPLES

Now, the present invention is described with reference to examples in detail.

### Example 1

A mixture of 51.4 g of sodium acetate, 150 ml of water, and 500 ml of acetonitrile was stirred at room temperature, and 100.7 g of 3-methoxy-3-iminoethylpropionate hydrochloride was added thereinto, followed by stirring for 5 min. Then, 96 g of 4-tert-butylbenzoic acid hydrazide was added thereto, followed by stirring at 15 °C for 2 hours. Then 850 ml of water and 106 g of sodium carbonate were added to the reaction system, and the reaction mixture was heated on a water bath under reflux. After 2 hours, the inside temperature was cooled with water to 30 °C, and 170 ml of concentrated hydrochloric acid was added, dropwise, to neutralize the reaction mixture. After the neutralization, the reaction liquid was cooled with ice, and the deposited crystals of 3-(4-tert-butylphenyl)-lH-1,2,4-triazol-5-yl acetic acid were filtered.

Obtained amount: 103.7 g; yield: 80%; melting point: 118 to 120 (decomposed).
¹H-NMR (CDCl₃) δ/ppm: 1.3 ppm (S. 9H), 4.18 (S. 2H), 7.4 to 7.55 (d. 2H), 7.8 to 8.0 (d. 2H)

### Example 2

A mixture of 26 g of 3-[(4-tert-butylbenzoyl)-hydrazino]-3-iminopropionic acid ethyl ester, 18.1 g of sodium carbonate, and 170 ml of water was heated and stirred, with the internal temperature being 80 °C. After 2 hours, the reaction liquid was cooled, and 29 ml of HCl was added, dropwise, to neutralize the reaction liquid, with the internal temperature being 30 °C. The deposited crystals were filtered, to obtain crystals of 3-(4-tert-butylphenyl)-1H-1,2,4-triazol-5-yl acetic acid. Obtained amount: 21 g; yield: 95%.

Specific examples of the compound represented by formula 4 of the present invention that is obtained in the above Steps 1 and 2 are shown below, but the present invention is not limited to them. 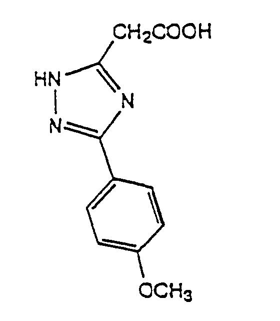 Melting point: 93 to 95 °C (decomposed) 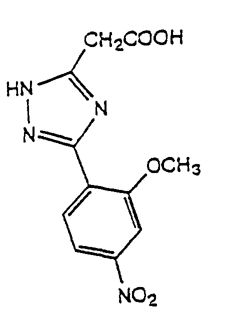 Melting point: 222 °C 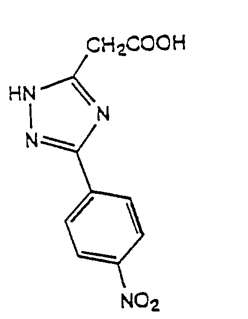 Melting point: 234 to 235 °C 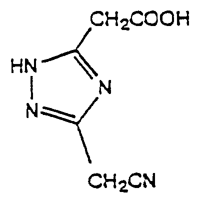 Melting point: 147 to 148 °C (decomposed)

## Claims

1. A method for preparing a 1H-1,2,4-triazol-5-yl acetic acid compound, comprising reacting a compound represented by the below-given formula (1), wherein R1 represents an alkyl group or aryl group, with a compound represented by the below-given formula (2), wherein R2 represents an alkyl group, R3 represents an alkyl group or aryl group, A represents an acid substance selected from the group consisting of HCl, HBr, HI, phosphoric acid, H₂SO₄, and p-toluenesulfonic acid, and n is 0, 0.5 or 1, in the presence of water, a water-soluble organic solvent, and CH₃COOM or a combination of NaOH and CH₃COOM, in which M represents an alkali metal, to produce a compound represented by the below-given formula (3), wherein R1 and R2 have the same meanings as those in formulas (1) and (2); and then adding M₂CO₃, wherein M has the same meaning as defined above, followed by heating, thereby obtaining a compound represented by the below-given formula (4),

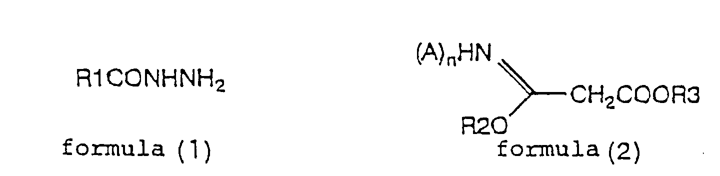

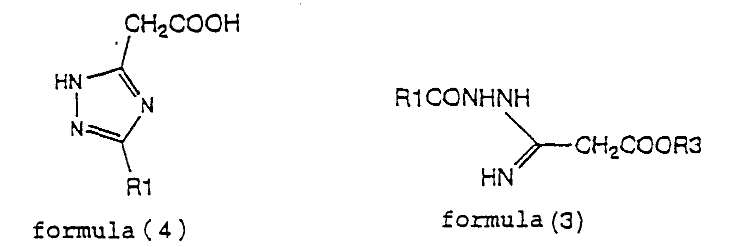

2. The method as claimed in claim 1, wherein the amount of at least one of NaOH and CH₃COOM used is, in a molar ratio to the compound of formula (1), 0.8 to 10 : 1.

3. The method as claimed in claim 1, wherein the amount of compound of formula (2) in a molar ratio to the compound of formula (1) is 0.8 to 1.5 : 1.

4. The method as claimed in claim 1, wherein the amount of M₂CO₃ used is, in a molar ratio to the compound of formula (1), 1 to 10 : 1.

5. The method as claimed in any one of claims 1 to 4, wherein 1H-1,2,4-triazol-5-yl acetic acid compound is a 3-(4-tert-butylphenyl)-1H-1,2,4-triazol-5-yl acetic acid compound.

6. A method for preparing a 1H-1,2,4-triazol-5-yl acetic acid compound, comprising heating a compound represented by formula (3), wherein R1 represents an alkyl group or aryl group and R3 represents an alkyl group or aryl group, in the presence of water and M₂CO₃, wherein M represents an alkali metal, to obtain a compound represented by formula (4), wherein R1 has the same meaning as defined in above formula (3).

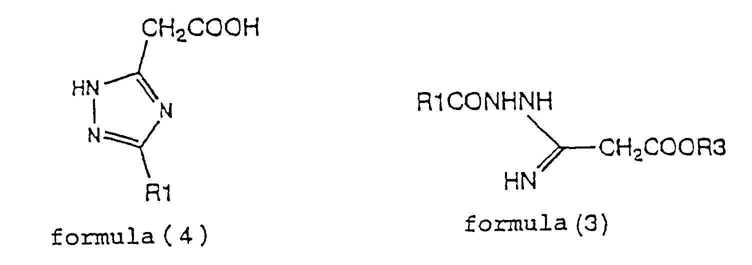

7. The method as claimed in claim 6, wherein the amount of M₂CO₃ used is, in a molar ratio to the compound of formula (3), 1 to 10 : 1.

## Patentansprüche

1. Verfahren zur Herstellung einer lH-1,2,4-triazol-5-yl-essigsäure-Verbindung, umfassend das Umsetzen einer durch die nachfolgend angegebene Formel (1) dargestellten Verbindung, worin R1 eine Alkyl-Gruppe oder Aryl-Gruppe darstellt, mit einer durch die unten angegebene Formel (2) dargestellten Verbindung, worin R2 eine Alkyl-Gruppe darstellt, R3 eine Alkyl-Gruppe oder Aryl-Gruppe darstellt, A eine Säuresubstanz darstellt, ausgewählt aus der Gruppe, die aus HCl, HBr, HI, Phosphorsäure, H₂SO₄ und p-Toluolsulfonsäure besteht, und n 0, 0,5 oder 1 ist, in Gegenwart von Wasser, eines wasserlöslichen organischen Lösungsmittels und CH₃COOM oder eine Kombination aus NaOH und CH₃COOM, wobei M ein Alkalimetall darstellt, um eine durch die nachfolgend angegebene Formel (3) dargestellte Verbindung zu erzeugen, worin R1 und R2 die gleichen Bedeutungen wie in den Formeln (1) und (2) haben; und dann Zugeben von M₂CO₃, worin M die gleiche Bedeutung wie oben definiert hat, gefolgt von Erwärmen, wodurch eine durch die nachfolgend angegebene Formel (4) dargestellte Verbindung enthalten wird:

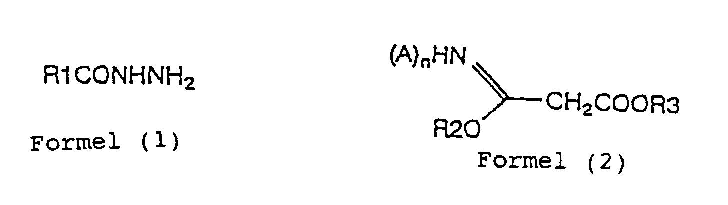

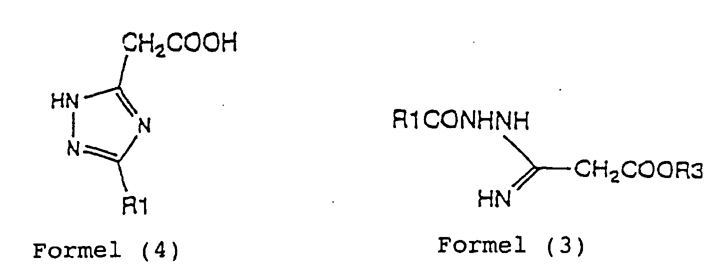

2. Verfahren gemäß Anspruch 1, worin die verwendete Menge wenigstens eines Vertreters aus NaOH und CH₃COOM als Molverhältnis zur Verbindung der Formel (1) 0,8 bis 10:1 ist.

3. Verfahren gemäß Anspruch 1, worin die Menge der Verbindung der Formel (2) als Molverhältnis zur Verbindung (1) 0,8 bis 1,5:1 beträgt.

4. Verfahren gemäß Anspruch 1, worin die verwendete Menge von M₂CO₃ als Molverhältnis zur Verbindung der Formel (1) 1 bis 10:1 beträgt.

5. Verfahren gemäß einem der Ansprüche 1 bis 4, worin die 1H-1,2,4-Triazol-5-yl-essigsäure-Verbindung eine 3-(4-tert-Butylphenyl)-1H-1,2,4-triazol-5-yl-essigsäure-Verbindung ist.

6. Verfahren zur Herstellung einer lH-1,2,4-Triazol-5-yl-essigsäure-Verbindung, umfassend das Erwärmen einer durch Formel (3) dargestellten Verbindung, worin R1 eine Alkyl-Gruppe oder Aryl-Gruppe darstellt und R3 eine Alkyl-Gruppe oder Aryl-Gruppe darstellt, in Gegenwart von Wasser und M₂CO₃, worin M ein Alkalimetall darstellt, um eine durch Formel (4) dargestellte Verbindung zu erhalten, worin R1 die gleiche Bedeutung wie in der obigen Formel (3) definiert hat.

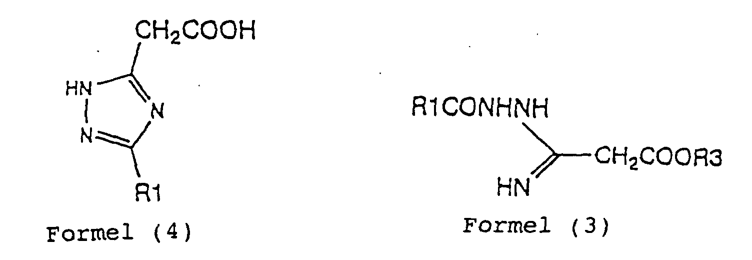

7. Verfahren gemäß Anspruch 6, worin die verwendete Menge von M₂CO₃ als Molverhältnis zur Verbindung der Formel (3) 1 bis 10:1 ist.

## Revendications

1. Procédé de préparation d'un composé d'acide 1H-1,2,4-triazol-5-yl-acétique, comprenant les étapes consistant à faire réagir un composé représenté par la formule (1) indiquée ci-dessous, dans laquelle R1 représente un groupe alkyle ou un groupe aryle, avec un composé représenté par la formule (2) indiquée ci-dessous, dans laquelle R2 représente un groupe alkyle, R3 représente un groupe alkyle ou un groupe aryle, A représente une substance acide choisie dans le groupe constitué par HCl, HBr, HI, l'acide phosphorique, H₂SO₄ et l'acide p-toluènesulfonique, et n est égal à 0, 0,5 ou 1, en présence d'eau, d'un solvant organique soluble dans l'eau, et de CH₃COOM ou d'une combinaison de NaOH et de CH₃COOM, où M représente un métal alcalin, pour produire un composé représenté par la formule (3) indiquée ci-dessous, dans laquelle R1 et R2 ont la même signification que dans les formules (1) et (2) ; puis à ajouter du M₂CO₃, où M a la même signification que celle définie ci-dessus, avant de chauffer l'ensemble pour obtenir un composé représenté par la formule (4) indiquée ci-dessous,

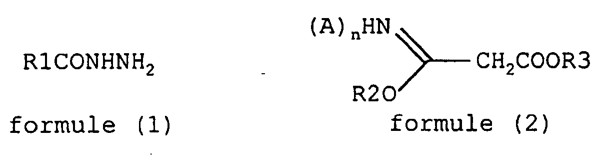

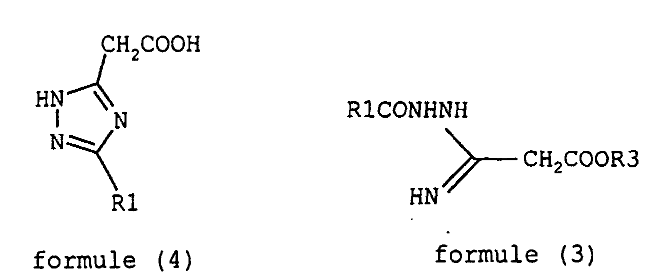

2. Procédé selon la revendication 1, dans lequel la quantité utilisée d'au moins un élément parmi NaOH et CH₃COOM est, dans un rapport molaire sur le composé de formule (1), de 0,8 à 10/1.

3. Procédé selon la revendication 1, dans lequel la quantité de composé de formule (2) dans un rapport molaire sur le composé de formule (1), est de 0,8 à 1,5/1.

4. Procédé selon la revendication 1, dans lequel la quantité de M₂CO₃ utilisée est, dans un rapport molaire sur le composé de formule (1), de 1 à 10/1.

5. Procédé selon l'une quelconque des revendications 1 à 4, dans lequel le composé d'acide 1H-1,2,4-triazol-5-yl-acétique est un composé d'acide 3-(4-tert-butylphényl)-1H-1,2,4-triazol-5-yl-acétique.

6. Procédé de préparation d'un composé d'acide 1H-1,2,4-triazol-5-yl acétique, comprenant l'étape consistant à chauffer un composé représenté par la formule (3), dans laquelle R1 représente un groupe alkyle ou un groupe aryle et R3 représente un groupe alkyle ou un groupe aryle, en présence d'eau et de M₂CO₃, où M représente un métal alcalin, pour obtenir un composé représenté par la formule (4), dans laquelle R1 a la même signification que celle définie dans la formule (3) ci-dessus.

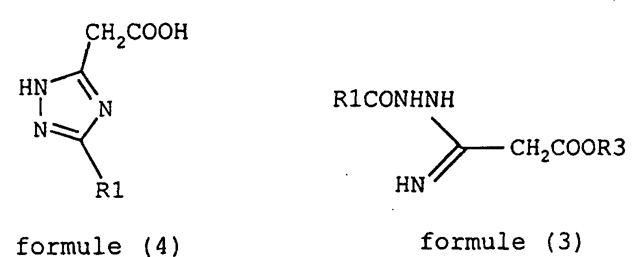

7. Procédé selon la revendication 6, dans lequel la quantité de M₂CO₃ utilisée est, dans un rapport molaire sur le composé de formule (3), de 1 à 10/1.
